Europäisches Patentamt

European Patent Office

Office européen des brevets

(11) Publication number: **0 506 147 A2**

# EUROPEAN PATENT APPLICATION

(21) Application number: **92109639.2**

(22) Date of filing: **17.02.88**

(51) Int. Cl.⁵: **A61K 39/175**

This application was filed on 09 - 06 - 1992 as a divisional application to the application mentioned under INID code 60.

(30) Priority: **09.03.87 US 23814**

(43) Date of publication of application: **30.09.92 Bulletin 92/40**

(60) Publication number of the earlier application in accordance with Art.76 EPC: **0 282 179**

(84) Designated Contracting States: **AT BE CH DE ES FR GB GR IT LI LU NL SE**

(71) Applicant: **DIAMOND SCIENTIFIC CO.**
**2538 S.E. 43rd Street**
**Des Moines Iowa 50317(US)**

(72) Inventor: **Bass, Edmund P.**
**1026 Cascade Drive**
**Menlo Park, California 94024(US)**
Inventor: **Kelsey, William H.**
**1201 Office Park Road**
**West Des Moines, Iowa 50265(US)**
Inventor: **McFarland, Michael D.**
**595 North Pleasant Hill Boulevard Apt. 120**
**Des Moines, Iowa 50317(US)**

(74) Representative: **Thomson, Paul Anthony**
**Potts, Kerr & Co. 15, Hamilton Square**
**Birkenhead Merseyside L41 6BR(GB)**

(54) **Canine distemper virus vaccine.**

(57) A vaccine composition for animals susceptible to infection by canine distemper virus is disclosed. The vaccine comprises an immunologically effective amount of an inactivated canine distemper virus in combination with a non-toxic pharmaceutically acceptable immunologic adjuvant, inactivation of the canine distemper virus being effected by exposure of the virus to long wavelength ultraviolet light ultraviolet radiation or gamma radiation in the presence of a furocoumarin.

Canine distemper is one of the most serious viral diseases of dogs. The disease is highly contagious and is characterised by sever morbidity and high mortality. Modified live canine distemper virus vaccines are currently available in the United States. They do provide protective immunity to distemper, but such vaccines may revert to virulence, may cause immunosupression following vaccination, and have been shown to cause mortality in non-canine species. There is therefore a real need for an efficacious, inactivated canine distemper virus vaccine, which eliminates the serious problems associated with modified live canine distemper virus vaccines.

An object of the present invention is to develop an inactivated canine distemper virus vaccine which substantially eliminates the serious problems, and risks associated with modified live canine distemper virus vaccines.

The present invention is directed to a new vaccine composition for canine distemper virus. It is also directed to a method for inactivating canine distemper virus, such that the virus while inactivated, nevertheless substantially retains its immunogenicity.

According to the present invention there is provided a vaccine composition for animals susceptible to infection by canine distemper virus (CDV) said composition comprising a small but immunologically effective amount of an inactivated canine distemper virus which although inactivated substantially retains its immunogenicity and a non-toxic pharmaceutically acceptable immunologic adjuvant, characterised in that the canine distemper virus is inactivated by an inactivation procedure comprising exposing a virus to an inactivating effective amount of long wave length ultraviolet light, ultraviolet radiation or gamma radiation, in the presence of a furocoumarin.

Also according to the present invention there is provided a process of inactivating canine distemper virus which allows the virus to substantially retain its immunogenicity, characterised in that it comprises exposing the virus to long wave length ultraviolet light, ultraviolet radiation or gamma radiation at a temperature below 40°C in the presence of an inactivating effective amount of a furocoumarin, for a time sufficient to render the virus non-infectious.

In this invention, inactivated vaccines are manufactured for the protection of mammalian species, in particular the canine, from severe clinical signs and mortality resulting from infection with canine distemper virus (CDV). The vaccines are inactivated preferably by combining virus fluids with a sufficient amount of a furocoumarin to provide for complete inactivation of CDV upon irradiation with long wavelength ultraviolet light (UVA), preferably while maintaining an inert atmosphere. The resulting inactivated virus preparation may be stored until used for vaccination.

Any strain of CDV may be inactivated for use in this invention. Of particular interest is the Rockborn strain, but other strains such as Snyder-Hill, Onderstepoort, etc., may be used.

In preparing the vaccine, virulent CDV is grown in cultured mammalian cells in conventional virus growth conditions. Examples of cells used are the DKF (dog kidney) cell line, and tertiary canine kidney cells. The host cells may be seeded with virus at the time of cell planting, or with a CDV-containing media change when the cell monolayer is 90-100% confluent. The multiplicity of infection (MOI) ratio may be from about 0.001 to about 0.05, preferably about 0.01. Any appropriate mammalian cell growth medium, such as Eagle's Minimum Essential Medium, containing bovine serum at from 0-10%, and antibiotics, is used to produce the viral fluids.

Infected cell cultures are maintained in a temperature range of from about 35°C to about 40°C for from 2 to about 7 days post seeding at which time virus-containing fluids are harvested. The CDV infected cultures may be refed with cell growth medium, which may be harvested after an additional 2 to 5 days incubation. Virus fluids are harvested into sterile containers and may be clarified by filtration. The virus fluids may further be concentrated, either prior to or post inactivation, using conventional ultrafiltration technology (e.g. Millipore Pellicon system XX42ASY60 with a cassette having a normal exclusion limit of $10^5$ daltons such as Millipore PTHK000C5).

The virulent canine distemper virus after harvest is ready for inactivation. The inactivation must be by a technique which destroys the virus' ability to replicate, but at the same time does not destroy the immunological activity of the inactivated virus. Heretofore there never has been a successful inactivated canine distemper virus vaccine. In this invention there is provided a virus which is both inactivated and at the same time retains immunogenicity.

As used herein, the term "inactivated" refers to previously virulent virus which have undergone treatment to inactivate, kill, or otherwise modify them to substantially eliminate their virulent properties while yet retaining their characteristic property of immunogenicity.

In accordance with this invention, the virus fluids may be inactivated by furocoumarin compounds. These compounds are primarily illustrated by the class of compounds referred to as psoralens, which includes psoralent and pharmaceutically acceptable derivatives thereof, where the substituents can be:

alkyl, preferably of from 1 to 3 carbon atoms, e.g., methyl, ethyl and propyl; alkoxy, particularly of from 1 to 3 carbon atoms e.g. methoxy, and substituted alkyl, of $C_1$ to $C_6$, preferably of $C_1$ to $C_3$ having from 1 to 2 heteroatoms, which can be oxy, particularly hydroxy or alkoxy of from 1 to 3 carbon atoms, e.g. hydroxymethyl and methoxymethyl; or amino, including mono- and dialkyl amino or amino-alkyl, having a total of from $C_1$ to $C_6$ carbon atoms, e.g. amino-methyl. There may be from 1 to 5, usually 2 to 4 substituents, which will normally be at the 4, 5, 8, 4' and 5' positions, particularly at the 4' position. Illustrative compounds include: 5-methoxypsoralen; 8-methoxypsoralen (8-MOP); 4, 5', 8-trimethylpsoralen (TMP); 4'-hydroxymethyl-4,5',8-trimethylpsoralen (HMT); 4'-aminomethyl-4,5', 8-trimethylpsoralen (AMT); 4-methylpsoralen; 4,4'-dimethylpsoralen; 4,5'-dimethylpsoralen; 4',8-dimethylpsoralen; and 4'-methoxy-methyl-4,5',8-trimethylpsoralen. For further details on suitable psoralens see Giles et al., US Patent 4,545,987 issued October 8, 1985, and Wiesehahn et al. US Patent 4,556,556 issued December 3, 1985.

The furocoumarins may be used individually or in combination. Each of the furocoumarins may be present in amounts ranging from 0.01 $\mu$g/m1 (micrograms/m1) to 1 mg/m1, preferably a lower level of from about 0.5 $\mu$g/m1, and most preferably there not being less than about 1 $\mu$g/m1 nor more than about 1 mg/m1 of furocoumarins.

In carrying out the process of this invention the furocoumarin(s), in an appropriate solvent which is substantially inert and sufficiently polar to allow for dissolution of the furocoumarin(s) are combined with the viral suspension, conveniently a viral suspension in an aqueous buffered medium, such as used for storage. The amount of virus will generally be about $1 \times 10^4$ to $10^8$, more usually about $1 \times 10^5$ to $10^7$ and preferably about $1 \times 10^{5.8}$ to $1 \times 10^{6.2}$ $CCID_{50}$/m1 (Cell Culture Infectious Dose). The amount of solvent which is used to dissolve the furocoumarin should be sufficiently small so as to readily dissipate in the aqueous viral suspension and have little, if any, effect on the results.

The psoralen(s) may be added to the viral suspension in a single addition or in multiple additions, where the virus is irradiated between additions. Usually, the number of additions can be from about 1 to about 5, more usually from about 1 to about 4, and preferably from about 2 to about 4. The total amount of psoralen(s) which can be added should be sufficient to provide a concentration of at least about 0.01 mg/m1 to about 1 mg/m1, usually not more than about 0.75 mg/m1 and preferably not more than about 0.5 mg/m1. Since a proportion of the psoralen(s) will have reacted with the canine distemper virus RNA between additions, the total concentration in solution will generally not exceed about 0.3 mg/m1.

The total time for the irradiation will vary depending upon the light intensity, the concentration of the psoralen(s), the concentration of the virus, and the manner of irradiation of the virus, where the intensity of the irradiation may vary. The total time will usually be at least from about 10 minutes to not more than about 60 hours, generally ranging from about 2 hours to 50 hours. The times between additions of for example psoralen, where the psoralen is added incrementally, will generally vary from about 1 hour to 24 hours, more usually from about 2 hours to 20 hours.

The temperature for the irradiation is preferably under 25°C, more preferably under 20°C, and will generally range from about -10° to about 15°C, more usually from about 0° to about 10°C.
The irradiation is normally carried out in an inert atmosphere, where all or substantially all of the air has been removed. Inert atmospheres include nitrogen, helium, argon, etc.

The light which is employed will generally have a wavelength in the range from about 300 nm to 400 nm. The intensity will generally range from about 0.1 mW/cm$^2$ to about 10 W/cm$^2$.

Optionally, a small amount of a singlet oxygen scavenger may be included during the virus inactivation. Singlet oxygen scavengers include ascorbic acid, dithioerythritol, sodium thionite, glutathione, superoxide dismutase etc. The amount of scavenger will generally be at a concentration of about 0.001 M to 0.5 M, more usually at about 0.05 M to 0.2 M, where the addition may be made in a single or multiple additions.

During irradiation, the medium may be maintained still, stirred or circulated and may be either continuously irradiated or be subject to alternating periods of irradiation and non-irradiation. The circulation may be in a closed loop system or in a single pass system ensuring that all of the sample has been exposed to irradiation.

It may be at times desirable to remove the unexpended furocoumarin and/or its photo-breakdown products from the irradiation mixture. This can be readily accomplished by one of several standard laboratory procedures such as dialysis across an appropriately sized membrane or through an appropriately sized hollow fiber system after completion of the irradiation. Alternatively, one may use affinity columns for one or more of the low molecular weight materials to be removed.

Either of the above preferred inactivating procedures (i.e. BEI or furocoumarin technique) may be employed with satisfactory results. However, it is preferred that one employ the technique of use of furocoumarins coupled with irradiation, since psoralen inactivated CDV induces higher serum neutralisation antibody titers in vaccinated dogs, compared to BEI inactivated CDV vaccine.

It is also possible to use other inactivating techniques, such as the use of ultraviolet radiation or gamma radiation.

In preparation of the vaccine, the inactivated collected canine distemper virus is mixed with a non-toxic pharmaceutically acceptable immunologic adjuvant such as Freund's complete adjuvant, aluminum hydroxide or oil-in-water or water-in-oil adjuvants. Also, Interlukin-1, or other immuno-enhancing substances may be used. Preparation of the vaccine entails mixing the inactivated virus fluids with an adjuvant. The amount of virus per dose of vaccine is equivalent to from approximately $10^4$ to $10^8$ 50% cell culture infective doses per ml $CCID_{50}/m1$. In addition, other viruses or bacteria may be included such as, but not limited to, canine adenovirus type 2, canine parainfluenze virus, canine parvovirus, Leptospira canicola and Leptospira icterohaemorrhagiae. The vaccine may be administered subcutaneously or intramuscularly. The vaccine inoculation volume will range from about 0.5 m1 to 4 m1. Normally 2 injections are given at from 1 to 3 week intervals.

Preferably the dosage of the inactivated canine distemper virus is from about $10^4$ to about $10^8$ $CCID_{50}/m1$, that is, cell culture infective dose/m1. More preferably, within the range of from about $10^5$ to about $10^7$ $CCID_{50}/m1$ and most preferably within the range of about $10^{5.8}$ to about $10^{6.2}$ $CCID_{50}/m1$.

The following examples are offered to further illustrate but not limit both the composition of the invention, the method of preparation of the composition of the invention, and the method of use of the composition of the invention to immunise the mammals against canine distemper virus.

## EXAMPLES

Example 1

Virus Growth, Assay and Inactivation

This example illustrates how the canine distemper virus was grown, assayed, and inactivated using a furocoumarin inactivation technique.

## A. Production of Virus and Tissue Culture

Canine cells (DKF), a dog kidney cell line, or (DK) tertiary dog kidney cells are grown as monolayers in plastic cell culture vessels in Eagle's Minimum Essential Medium with Earle's salts and non-essential amino acids (MEN) supplemented with 5% heat inactivated fetal bovine serum or 5% heat inactivated calf serum. Cell cultures are used to produce live CDV from master seed virus. Cells are grown in culture vessels to 80% to 100% confluency (approximately $2 \times 10^5$ cells per $cm^2$ of growth surface area) using standard mammalin cell culture techniques. Generally, plastic roller bottles (e.g. Corning No. 25140-850) with a growth surface area of 850 $cm^2$ containing 100 m1 of MEN supplemented with 5% fetal bovine serum and 1 $\times 10^8$ to $2 \times 10^8$ cells/bottle are used for virus production although other permissive cells, other culture vessels, other culture media or other supplements may be used. The cell cultures are initiated by seeding approximately $1 \times 10^7$ to $2 \times 10^7$ cells into 100 mls of growth medium in a roller bottle on a roller bottle rotator at 0.25 to 2 rpm at 35° to 38°C. The cultures are grown to 80% to 100% confluency over a seven to ten day period with a medium change every three to five days.

When the monolayers are 80% to 100% confluent the culture medium is removed. Two hundred mls of MEN supplemented with 0.5% lactalbumin hydrolysate (e.g. GIBCO 670-1800) is added per roller bottle. Monolayers are infected with $10^5$ to $10^6$ $CCID_{50}$ of CDV per roller bottle. The multiplicity of infection (MOI) is approximately 0.01. The MOI may range from 0.001 to 0.05. The post-infection incubation is at 35° to 38°C with rotation. Two to five days post-infection, CDV cytopathic effect (CPE) is evident. Two days post-infection, the CPE is characterised by the appearance of some vacuolated cells and some multi-nucleated giant cells. On day five post-infection, giant cells are more numerous. On day five post-infection, the medium containing virus is harvested from the roller bottle. The roller bottle is fed 200 ml fresh MEN supplemented with 0.5% LAH and incubated at 35° to 38°C with rotation for an additional three days. During the six to eight day post-infection period, the CPE progresses to the point that many cells are shed into the medium. By day eight post-infection, 50% to 90% CPE is evident. On day eight post-infection, the medium containing virus is harvested.

The virus preparation may be concentrated by ultra-filtration using a Pellicon cassette system (Millipore XX42ASY60) with a cassette having a nominal exclusion of $10^5$ daltons (Millipore PTHK000C5). The Pellicon cassette system is sterilised by filling the assembled unit with 1 N NaOH and incubating the unit 12 to 14 hours at room temperature. The NaOH solution is pumped out of the cassette system and the system is

4

flushed with two to four litres of sterile $H_2O$. The assembly and operation of the Pellicon system are in accordance with the instruction furnished by the manufacturer. All steps in the concentration are performed aseptically.

## B. Virus Assay

Ten-fold serial dilutions of a virus sample are made by adding 0.1 ml of the virus sample to 0.9 ml of MEN supplemented with 5% $F^i$. Eight chamber Lab-Tek slides are seeded with 0.4 ml/chamber of DKF cells at $1.5 \times 10^5$ cells/ml in MEN supplemented with 5% $F^i$ (approximately $6 \times 10^4$ cells/chamber). Immediately following seeding of cells, 0.1 ml aliquots of each serial dilution are added to each of five chambers. The slides are incubated at 35° to 38°C in 5% $CO_2$ in air for six days. On day six, remove the medium, plastic chamber and gasket from the slide. Rinse slides once in PBS, fix in acetone at -20°C for 20 minutes and air dry. Anti-CDV FITC conjugate (0.1ml) is placed on each slide and a coverslip is placed on the monolayer to spread the conjugate over the entire cell sheet. Slides are incubated in a high humidity incubator for 30 minutes at 35° to 37°C. Coverslips are removed in 3x3 minute rinses in PBS at room temperature. Slides are counter stained for 90 seconds in Evans blue, rinsed twice for 3 minutes in PBS, rinsed in distilled water and placed in a modified X-ray dryer until dry. Coverslips are mounted on slides using mounting fluid 80% glycerol in PBS, v/v). Cells are examined for fluorescence typical of CDV which constitutes a positive response. End point is calculated by the method of Reed and Muench.

## C. CDV Inactivation

1. Psoralen Inactivation:

One hundred ml of CDV is pipetted into a sterile container. One ml of 8-methoxypsoralen (8-MOP, 10 mg/ml in dimethyl sulfoxide) and one ml of ascorbate stock solution (1M ascorbate in sterile de-ionized $H_2O$) are added to the container. The mixture is incubated 15 minutes in a 37°C water bath. After incubation the mixture is transferred to another sterile vessel and allowed to equilibrate in an argon atmosphere for 5 minutes. After equilibration the mixture is placed under a long wavelength ultraviolet light source at a temperature of 10°C for 2 hours. The incident light intensity is approximately 15 $mW/cm^2$.

After the irradiation is completed, the mixture is removed from the light source and an additional 1 ml of 8-MOP stock solution and 1 ml of ascorbate stock solution is added to virus. The mixture is again incubated 15 minutes in 37°C water bath. After incubation the mixture is transferred to sterile vessel and allowed to equilibrate in an argon atmosphere for 5 minutes and irradiated as described above for an additional 2 hours. This procedure is repeated one more time until three additions (a total of approximately 300 micrograms/ml) of 8-MOP have been performed and the virus sample has been irradiated for at least six hours.

The inactivated virus is now ready for use in preparation of vaccine. While the inactivation procedure described here is the preferred furocoumarin procedure, specifically using 8-methoxypsoralen, other inactivation procedures as mentioned earlier may be used.

## Example 2

## Challenge Test of Vaccinated Dogs

Twenty-two dogs seronegative to CDV were vaccinated intramuscularly (IM) with a psoralen-inactivated CDV vaccine prepared as in Example 1, given in two 1 ml doses, 21 days apart. The vaccine was formulated with 50% Adjuvant B. On experimental day 35, vaccinated and 5 nonvaccinated seronegative dogs were challenged with virulent CDV.

Serum samples for serologic evaluation were collected from vaccinated dogs on days 0, 21 and 35. Geometric mean titers are shown in Table I, demonstrating successful immunisation.

Clinical signs were not observed in vaccinated dogs following vaccination. Dogs were challenged by oral nasal installation of $10^6/CCID_{50}$ of virulent canine distemper vaccine. Following challenge, vaccinated and control dogs were observed for 21 days for clinical signs of disease which were assigned points using a clinical reaction index (Table II).

The clinical reaction scores for vaccinated and control group dogs are summarised in Table III. Four of five control dogs experienced severe illness, including one fatal infection, in contrast to vaccinated dogs in which clinical signs were, at most, mild and transient. This demonstrates the efficacy of the inactivated CDV

5

vaccine.

Table I

| RECIPROCAL GEOMETRIC MEAN VIRUS NEUTRALIZATION TITERS FROM DOGS VACCINATED WITH A PSORALEN-INACTIVATED CDV VACCINE | | | |
|---|---|---|---|
| Group | Day of Test | | |
| | 0 | 21 | 35 |
| I - Vaccinates (n = 22) | <2 | <6 | 139 |
| II - Controls | ND* | $<2^a$ | $<2^b$ |
| *ND = Not Done | | | |
| $^a$n = 5 | | | |
| $^b$n = 4 | | | |

Table II

| CANINE DISTEMPER VACCINE - PSORALEN-KILLED VIRUS KEY TO SCORING CLINICAL OBSERVATIONS | | |
|---|---|---|
| Anorexia (Q): | 1-2 days = <br> ≧ 3 days = | 1 pt <br> 2 pts |
| Dehydration (DH): | 1-2 days <br> ≧ 3 days | 1 pt <br> 2 pts |
| Depression (DP): | 1-2 days = <br> 3-5 days = <br> ≧ 6 days = | 2 pts <br> 4 pts <br> 6 pts |
| Diarrhea (B): <br> Bloody Diarrhea (BB): | each day = <br> each day = | 2 pts <br> 3 pts |
| Eye Discharge-Serous (CS): | 1-3 days = <br> ≧ 4 days = | 1 pt <br> 2 pts |
| Eye Discharge -Mucopurulent (CM or CP): | 1-2 days = <br> 3-5 days = <br> ≧ 6 days = | 2 pts <br> 4 pts <br> 6 pts |
| Nasal Discharge - Purulent (PP): | 1-2 days = <br> 3-5 days = <br> ≧ 6 days = | 2 pts <br> 4 pts <br> 6 pts |
| Weakness | 1-2 days = <br> ≧ 3 days = | 1 pt <br> 2 pts |
| Death due to distemper challenge | | 30 pts |

TABLE III

| SUMMARY OF CUMULATIVE CLINICAL OBSERVATION SCORES POST CHALLENGE | | |
|---|---|---|
| Group | Mean Clinical Score | Range |
| 1 - Vaccinates (n = 22)<br>2 - Controls (n = 5) | 0.95<br>30.8 | 0-5<br>2-53 |

The above methods for assaying vaccine virus and challenge testing demonstrates both preparation and successful use of the canine distemper vaccine of this invention. It also represents the first ever efficacious successful inactivated canine distemper vaccine. It therefore can be seen that the invention accomplishes at least all of its stated objectives.

In our copending Application No. 88301296.5, Publication No. 0282179, out of which the present application has been divided, there is disclosed a vaccine composition for animals susceptible to infection by canine distemper virus (CDV), said composition comprising a small but immunologically effective amount of an inactivated canine distemper virus which, although inactivated, substantially retains its immunogenicity and a non-toxic pharmaceutically acceptable immunologic adjuvant, characterized in that the canine distemper virus is inactivated by an inactivation procedure comprising exposure of the virus to an inactivating effective amount of binary ethyleneimine, acetyl ethyleneimine, beta-propriolactone, formalin or phenol. There is also disclosed a method for preparing such virus and the use of such composition for protecting mammals from infection caused by canine distemper virus.

**Claims**

1.  A vaccine composition for animals susceptible to infection by canine distemper virus (CDV) said composition comprising a small but immunologically effective amount of an inactivated canine distemper virus which although inactivated substantially retains its immunogenicity and a non-toxic pharmaceutically acceptable immunologic adjuvant, characterised in that the canine distemper virus is inactivated by an inactivation procedure comprising exposing a virus to an inactivating effective amount of long wave length ultraviolet light, ultraviolet radiation or gamma radiation, in the presence of a furocoumarin.

2.  A vaccine composition as claimed in claim 1, characterised in that the amount of the inactivated canine distemper virus is from $10^4$ to $10^8$ $CCID_{50}$/ml.

3.  A vaccine composition as claimed in claim 2, characterised in that the amount of the virus is from $10^5$ to $10^7$ $CCID_{50}$/ml.

4.  A vaccine composition according to claim 3, characterised in that the amount of the virus is from $10^{5.8}$ to $10^{6.2}$ $CCID_{50}$/ml.

5.  A vaccine composition according to any preceding claim, characterised in that the adjuvant comprises Freund's complete adjuvant, aluminum hydroxide or oil-in-water or water-in-oil adjuvants.

6.  A vaccine composition according to any preceding claim, characterised in that the canine distemper virus is the Rockborn, Snyder-Hill or Onderstepoort strain.

7.  A process of inactivating canine distemper virus which allows the virus to substantially retain its immunogenicity, characterised in that it comprises exposing the virus to long wave length ultraviolet light, ultraviolet radiation or gamma radiation at a temperature below 40°C in the presence of an inactivating effective amount of a furocoumarin, for a time sufficient to render the virus non-infectious.

8.  A process according to claim 7, characterised in that the amount of the virus is from $10^4$ to $10^8$ $CCID_{50}$/ml.

9.  A process according to claim 8, characterised in that the amount of the virus is from $10^5$ to $10^7$ $CCID_{50}$/ml.

**10.** A process according to claim 8, characterised in that the amount of the virus is from $10^{5.8}$ to $10^{6.2}$ $CCID_{50}$/ml.

**11.** A process according to any one of claims 7 to 10, characterised in that a mixture of two or more furocoumarin compounds are used.

**12.** A process according to claim 11, characterised in that each of the furocoumarins is present in an amount ranging from 0.01 $\mu$g/ml to 1.0 mg/ml.

**13.** A process according to any one of claims 7 to 12, in which the furocoumarin is a psoralen or a pharmaceutically acceptable derivative thereof.

**14.** A process according to any one of claims 7 to 13 characterised in that the exposure to ultraviolet light is carried out in the presence of a single oxygen scavenger.

**15.** A process according to any one of claims 7 to 14, characterised in that the canine distemper virus is grown in cultured mammalian host cells prior to inactivation.

**16.** A process according to any one of claims 7 to 15, characterised in that the temperature at which the inactivation occurs is within the range of from -10°C to 15°C.

**17.** A process according to any one of claims 7 to 16 characterised in that the exposure to irradiation by long wave ultraviolet light is in the presence of an inert atmosphere thus substantially excluding free oxygen.

**18.** The use of a vaccine composition according to any of claims 1 to 6 for the manufacture of a medicament for protecting mammals from infection caused by canine distemper virus.